(19) Europäisches Patentamt
European Patent Office
Office européen des brevets

(11) Publication number: **0 485 664 A1**

(12) **EUROPEAN PATENT APPLICATION**

(21) Application number: **90202996.6**

(51) Int. Cl.[5]: **A61K 49/02**

(22) Date of filing: **13.11.90**

(43) Date of publication of application:
**20.05.92 Bulletin 92/21**

(84) Designated Contracting States:
**NL**

(71) Applicant: **Mallinckrodt Diagnostica (Holland) B.V.**
**Westerduinweg 3**
**NL-1755 LE Petten(NL)**
Applicant: **ACADEMISCH ZIEKENHUIS LEIDEN**
**Rijnsburgerweg 10**
**NL-2333 AA Leiden(NL)**

(72) Inventor: **Calame, Wim,**
**c/o OCTROOIBUREAU ZOAN B.V. P.O. Box 140**
**NL-1380 AC Weesp(NL)**
Inventor: **Feitsma, Hans I.J.**
**c/o OCTROOIBUREAU ZOAN B.V. P.O. Box 140**
**NL-1380 AC Weesp(NL)**
Inventor: **Ensing, Geert J.**
**c/o OCTROOIBUREAU ZOAN B.V. P.O. Box 140**
**NL-1380 AC Weesp(NL)**
Inventor: **Pauwels, Ernest K.J.**
**c/o OCTROOIBUREAU ZOAN B.V. P.O. Box 140**
**NL-1380 AC Weesp(NL)**

(74) Representative: **Swaters, Pieter D. et al**
**OCTROOIBUREAU ZOAN B.V. P.O. Box 140**
**NL-1380 AC Weesp(NL)**

(54) **Method of preparing a diagnostic agent for detecting inflammations.**

(57) The invention relates to a method of preparing a diagnostic agent for detecting and locating inflammations in a warm-blooded living being by attaching a detactable label to an immunospecific protein or proteinaceous substance, wherein prior to the labelling procedure the protein or proteinaceous substance is purified with the aid of a substance comprising immunoglobulin receptors.

The invention further relates to a kit comprising said purified protein or proteinaceous substance.

EP 0 485 664 A1

The invention relates to a method of preparing a diagnostic agent for detecting and locating inflammations in a warm-blooded living being by attaching a detectable label to an immunospecific protein or proteinaceous substance. The invention further relates to a diagnostic agent obtained by using said method, to a pharmaceutical composition comprising said diagnostic agent, to the use of said composition and to a kit for preparing said pharmaceutical composition.

Many physical complaints are caused by inflammations in the body. In order to be able to achieve a specific therapy, the detection and location of the inflammation site(s) in an early stage is of the utmost importance. A good diagnostic agent is also indispensable for supporting the therapy used. Various requirements have to be imposed on such a diagnostic agent, for example, non-toxic, no adverse influence on the host resistance and/or therapeutic treatment, well detectable and selective. The required high selectivity means that the diagnostic agent, after having been introduced into the body, must accumulate selectively at the site of the inflammation to be detected. In order to be detectable from outside the body, the diagnostic agent should be labelled, preferably with a radionuclide or with a paramagnetic metal isotope. In the former case, the radioactive radiation can be detected by using a suitable detector (scanning). Modern techniques in this field use emission tomography; when gamma radiating isotopes are used, the so-called single photon emission computerized tomography (SPECT) may be applied. The use of paramagnetic diagnostic agents enables a detection by means of imaging by magnetic resonance (magnetic resonance imaging).

European Patent Application 241106 describes the use of labelled immunoglobulins or fragments thereof for diagnosing inflammations. As is stated in said patent specification, immunoglobulins tend to accumulate more strongly in the inflamed sites than in the non-inflamed sites. As a result of this selectivity, i.e. a comparatively stronger concentration at the site of the inflammation, labelled immunoglobulins may be used for diagnostic imaging. From the examples of said patent specification a notable preference of radioactive-labelled immunoglobulins indeed appears for accumulation in inflamed sites over uptake in non-inflamed sites of the body.

However, the selectivity, i.e. the specific accumulation of the above-described labelled immunoglobulins in the inflamed sites, is still unsatisfactory in practice. Selectivity is to be understood to mean the target-to-non-target ratio. This means that a comparatively large radioactivity is found in the non-target tissues, e.g. in the blood, as a result of which the image of the inflammation to be examined is obscured. Moreover, when a considerable quantity of radioactivity remains circulating, the locating of inflammations is disturbed in case these are present in certain sites of the body. As a result of this a good imaging, in particular in an early stage of the inflammation, is disturbed so that a correct diagnosis is impeded. Therefore there exists a need for agents for diagnosing inflammations with a better selectivity than the above-described labelled immunoglobulins known for this purpose.

It has now been found surprisingly that said need can be met by using a protein or proteinaceous substance, which prior to the labelling procedure has been purified with the aid of a substance comprising immunoglobulin receptors. Consequently the present invention relates to a method of preparing a diagnostic agent as defined in the opening paragraph, and is characterized in that prior to the labelling procedure the protein or proteinaceous substance is purified with the aid of a substance comprising immunoglobulin receptors.

Suitable immunospecific proteins and proteinaceous substances which can be used as starting materials for the method of the invention are, in particular, exogenous proteins or fragments thereof, such as human immunoglobulins or suitable fragments thereof, glycopeptides, oligopeptides, monoclonal antibodies such as antimyosin and other proteins suitable for the purpose in view, such as plasmin and tissue plasminogen activator. The use of human proteins instead of animal proteins is advantageous in that no detrimental antibodies, e.g. human anti-mouse-antibodies, are formed after intravenous administration in a human being.

Suitable substances comprising immunoglobulin receptors are, for example, protein-A. Such substances are preferably derived from gram-positive micro-organisms having a considerable number of immunoglobulin receptors, such as most Staphylococcus aureus species, Sarcina lutea, etc.

The use of protein-A containing materials for the quality control of proteins, intended for clinical use in patients with antibody deficiencies, is known in the art, e.g. from Le Moli et al, J. Clin. Lab. Immunol. 29, 1989, 79-84. The purification of human immunoglobulin as described above with the intention of achieving a more selective and specific diagnostic agent after labelling, however, is hitherto unknown in the art.

According to a preferred procedure, the purification is carried out by immobilizing the substance comprising bacterial immunoglobulin receptors on a suitable substrate, usual in such technics, such as sepharose, and by then subjecting a solution of the protein or proteinaceous substrate to be purified, preferably in a suitable aqueous buffer solution, to affinity chromatography. In the chromatographic process said substrate is used as

the immobile phase and the solution of the protein or proteinaceous substance as the mobile phase. The fraction of the protein or proteinaceous substance to be purified which has a high affinity to said substrate comprising the substance having immunoglobulin receptors is the fraction in question. By eluting the substrate with a suitable eluent under appropriate conditions the purified protein or proteinaceous substance can be isolated and then be labelled at each desired instant.

After purification as described above, the protein or proteinaceous substance should be labelled by attaching a detectable label thereto. The detectable label may be a radionuclide, preferably selected from the group consisting of I-123, I-131, Br-75, Br-76, Tc-99m, Pb-203, Ga-67, Ga-68, As-72, In-111, In-113m, Ru-97, Cu-62, Cu-64, Cu-67, Fe-52, Mn-52m and Cr-51, or a paramagnetic label, preferably selected from the group consisting of F-19, D, Na-23, P-31, Gd-157, Mn-55, Dy-162, Cr-52 and Fe-56. The label may be attached to the purified protein or proteinaceous substance directly, if desired after a reductive treatment of said protein or proteinaceous substance, or through a suitable linker.

Direct labelling of the protein with radioactive halogen can be carried out by reacting the protein with a suitable compound of the radionuclide in a preferably aqueous solution, if desired in the presence of an oxidant. Suitable radioactive halogen compounds are radioactive alkali halogenides or radioactive halo-substituted aromates, such as tyrosine.

The direct labelling of the protein with a metal radionuclide has two disadvantages, via (i) that the biologically active site of the protein needed for a good selectivity can easily be blocked by said reaction so that the normal behaviour of the biologic macromolecule is disturbed, and (ii) that the affinity between metal isotope and macromolecule is often insufficient as a result of which the formed bond is insufficiently stable to remain intact under physiological conditions. Therefore in that case a reductive treatment of the protein, as suggested e.g. in European patent applications 237150 and 271806, is preferred. In such a pretreatment the disulphide bonds in the protein are reduced with a suitable agent, e.g. dithiothreitol, dithioerythritol, 2-mercaptoethanol or 2-mercaptoethylamine, after which the reduced protein, now comprising free mercapto groups, may be reacted with a salt or chelate of the desired metal isotope. Stabilization of the reduced protein or the final complex may be desired. It is generally recognized, however, that the reductive pretreatment of the protein may exert an unfavourable influence on the biological behaviour of the protein.

To avoid this disadvantage, it is proposed in the non-prepublished Netherlands patent application 9001137 in the name of Academisch Ziekenhuis Leiden to use an effective amount of a borohydride in the labelling with technetium-99m, in order to obtain a Tc-99m labelled protein or proteinaceous substance which is excellently suitable for the purpose in view. Equally suitable is the use of a suitable linker or coupling agent for preparing a metal-radionuclide-labelled protein or proteinaceous substance, e.g. as described in the Intern. patent application (PCT) WO 89/07456 and the European patent application 178125, both in the name of Mallinckrodt Inc. The protein or proteinaceous substance to be labelled is then first modified by treating with a coupling agent, after which the resulting protein conjugate is brought into a complexing reaction with a salt or chelate of the desired metal isotopes. In literature various different coupling agents are described, via compounds which after coupling with the protein can complex the metal isotope by an $N_2S_2$-, $N_3S$- or $N_4$- tetradentate ring structure, N-containing di- or polyacetic acids or their derivatives such as EDTA, DTPA, NTA, etc., amino-containing compounds such as the maleimide derivatives disclosed in the above EP 178125, peptide-derivatives, and compounds comprising chelating groups such as isocyanate, formyl, diazonium, isothiocyanate, alkoxycarbimidoyl groups and the like. For the modification of the protein or proteinaceous substance, purified according to the invention, however, a coupling agent is preferred which is described in the above WO 89/07456 and which may generally be represented by the formula

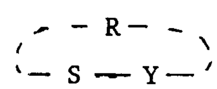

wherein

R is a branched or non-branched, optionally substituted hydrocarbyl radical, which may be interrupted by one or more hetero-atoms selected from N, O and S and/or by one or more NH groups, and

Y is a group which is capable of reacting with a functional group of the protein and which is preferably selected from the group consisting of carbonyl, carbimidoyl,

N-($C_1$-$C_6$) alkylcarbimidoyl, N-hydroxycarbimidoyl and N-($C_1$-$C_6$) alkoxycarbimidoyl, or a water-soluble salt of this ring compound. Examples of suitable coupling agents described in said Intern. patent application are substituted or unsubstituted 2-iminothiolanes and 2-iminothiacyclohexanes.

The modification of the protein or proteinaceous substance, i.e. the reaction with the coupling agent resulting in the protein conjugate, can generally be carried out in a simple manner. In the subsequent complex forming reaction, the metal isotope is presented to the protein conjugate in the form of a salt or chelate. In the latter case relatively weak chelators are used, e.g. a phosphonate or polyphosphonate, an oxinate, a carboxylate, a hydroxycarboxylate, an aminocarboxylate or an enolate. Then the desired complex is formed by ligand exchange. The complex forming reactions can generally be carried out in a simple manner and under conditions which spare the protein or proteinaceous substance.

The above-mentioned labelling with Tc-99m in the presence of a borohydride can equally be carried out in a simple manner and under very moderate conditions, e.g. at room temperature.

The invention further relates to a diagnostic agent obtained by using the method as defined above and to a pharmaceutical composition which comprises, in addition to a pharmaceutically acceptable carrier, a diagnostic agent obtained as defined above. Such a pharmaceutical composition is intended for diagnostic application. If desired the composition so obtained can be brought into a form more suitable for intravenous or subcutaneous application, e.g. by adding a pharmaceutically acceptable liquid carrier material. For intravenous or subcutaneous application the solution should of course be in a sterile condition.

For performing a diagnostic examination the composition, as described above, if desired after dilution with a pharmaceutically acceptable liquid, preferably a physiological saline solution, can be administered to a warm-blooded living being in a quantity sufficient for externally imaging said being to detect and locate an inflammation in said being. In case a radioactive labelled protein or proteinaceous substance is used as a diagnostic agent, the radioactive material is generally administered to the living being in a quantity from 1 to 2000 MBq, preferably from 100 to 1200 Mbq, per 70 kg of body weight. Thereupon the being is subjected to external imaging to detect accumulated radioactivity and thus to determine the location thereof in the body of the being.

In case a radioactive labelled protein or proteinaceous substance is used as a diagnostic agent, it is frequently impossible to put the ready-for-use composition at the disposal of the user, in connection with the often poor shelf life of the radiolabelled compound and/or the short half-life of the radionuclide used. In such cases the user will carry out the labelling reaction with the radionuclide in the clinical hospital or laboratory. For this purpose the various reaction ingredients are then offered to the user in the form of a so-called "kit". It will be obvious that the manipulations necessary to perform the desired reaction should be as simple as possible to enable the user to prepare from the kit the radioactive labelled composition by using the facilities that are at his disposal. Therefore the invention also relates to a kit for preparing a radiopharmaceutical composition.

Such a kit according to the present invention may comprise (i) an immunospecific protein or proteinaceous substance purified as described above, to which substance, if desired, an inert pharmaceutically acceptable carrier and/or formulating agents and/or auxiliary substances is/are added, (ii) a solution of a compound of a radionuclide, and (iii) instructions for use with a prescription for reacting the ingredients present in the kit. If in such a kit the radionuclide is radioactive halogen, preferably a halogen compound as defined hereinbefore is used as an ingredient of the kit.

Alternatively, such a kit according to the present invention may comprise (i) a substance obtained by treating a human immuno-specific protein or proteinaceous substance, purified as described above, with a suitable disulphide-reducing agent as defined hereinbefore, to which substance, if desired, an inert pharmaceutically acceptable carrier and/or formulation agents and/or auxiliary substances is/are added, (ii) a solution of a salt or chelate of a metal-radionuclide, and (iii) instruction for use with a prescription for reacting the ingredients present in the kit. A suitable carrier is a physiological saline solution. Examples of auxiliary substances are stabilizers, antioxidants and filling agents. Suitable metal-radionuclides have been mentioned hereinbefore. As stated above, the desired radionuclide for this complex forming reaction prescribed may be presented to the reduced protein in the form of a chelate, bound to a comparatively weak chelator, in which the reaction may take place in a neutral environment, e.g. a buffered substantially aqueous solution. The kit to be supplied to the user may also comprise the ingredient(s) defined sub (i) above, together with instructions for use, whereas the solution of the salt or chelate of the radionuclide, defined sub (ii) above, which solution has a limited shelf life, may be put to the disposal of the user separately.

In case the kit serves to prepare a radiopharmaceutical composition labelled with technetium-99m, such a kit according to the present invention may comprise, in addition to the ingredient(s) defined sub (i) above, (ii) a pertechnetate-reducing agent and, if desired, a chelator, and (iii) instructions for use with a prescription for reacting the ingredients of the kit with technetium-99m in the form of a pertechnetate solution. If desired, the ingredients of the kit may be combined, provided

they are compatible. The kit should comprise a reducing agent to reduce the pertechnetate, for example, a dithionite, a metallic reducing agent or a complex-stabilizing reducing agent, e.g. Sn(II)-tartrate, Sn(II)-phosphonate or -pyrophosphate, or Sn(II)-glucoheptonate. The pertechnetate can simply be obtained by the user from a suitable generator. Examples of suitable chelators have been described hereinbefore.

In a preferred embodiment the kit according to the present invention and intended for the preparation of a Tc-99m labelled composition comprises in addition a borohydride in a quantity effective for the labelling procedure.

In an equally preferred embodiment the kit according to the present invention comprises, instead of a reduced protein or proteinaceous substance, a protein conjugate, obtained by modifying an immunospecific protein or proteinaceous substance, purified as described hereinbefore, by a treatment with a coupling agent. Suitable coupling agents have been described hereinbefore. The use of a compound of the general formula

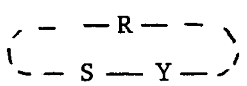

wherein the symbols have the meanings given hereinbefore, as a coupling agent is to be preferred.

When the radionuclide is present in the kit itself, the complex forming reaction with the protein conjugate can simply be produced by combining the components in a neutral medium and causing them to react. For that purpose the radionuclide is preferably presented to the protein conjugate in the form of a chelate bonded to a comparatively weak chelator. Examples of suitable chelators for the radionuclide are 8-hydroxquinoline or derivatives hereof; dicarboxylic acids, polycarboxylic acids or hydroxycarboxylic acids, for example, oxalic acid, malonic acid, succinic acid, maleic acid, orthophtalic acid, malic acid, lactic acid, tartaric acid, citric acid, ascorbic acid, salicylic acid or derivatives of these acids; pyrophosphates; phosphonates or polyphosphonates, for example, methylene diphosphonate, hydroxyethylene disphosphonate or hydroxymethylene diphosphonate; or enolates, for example, with a $\beta$-diketone, for example, acetyl acetone, furoyl acetone, thenoyl acetone, benzoyl acetone, dibenzoyl methane, tropolone or derivatives of these diketones. For this purpose are to be considered in particular 8-hydroxyquinoline, citric acid, tartaric acid, ascorbic acid, glucoheptonic acid or a derivative thereof, or acetyl acetone as chelators because it has been found that a chelate of a radionuclide, for example, indium-111 or lead-203, with one of these chelators in a suitable medium, preferably a buffered aqueous solution, easily reacts at a physiological pH with a protein conjugate as defined hereinbefore, the desired radionuclide complex being formed in a high yield and purity by ligand exchange. A buffered aqueous indium-111-tropolonate solution which may be used for the desired complex formation and is suitable for this purpose is described in European patent application no. 131327. The supplied kit may also consist of the constituent defined sub (1) with instructions for use, while the solution mentioned sub (2), which is bound to an expiration date, can be placed at the user' s disposal separately.

When the kit in addition comprises a borohydride, said borohydride is preferably $NaBH_4$ or $NaBH_3CN$. A quantity of 0.01-1 ug of borohydride is generally sufficient for labelling 0.1 mg of the protein or proteinaceous substance. Preferably also a pertechnetate-reducing agent is present in such a kit, e.g. Sn(II).

When the kit comprises a protein conjugate as defined hereinbefore and is intended for the preparation of a radiopharmaceutical composition, labelled with technetium-99m, the radionuclide will preferably be added separately in the form of a pertechnetate solution. In that case the kit will comprise a pertechnetate-reducing agent and, if desired, a chelator, the former to reduce the pertechnetate. As a pertechnetate-reducing agent may be used, for example, a dithionite or a metallic reducing agent. The ingredients may optionally be combined, provided they are compatible. Such a monocomponent kit, in which the combined ingredients are preferably freeze-dried, is excellently suitable for being reacted, by the user, with the radionuclide solution. As a reducing agent for the above-mentioned kits is preferably used a metallic reducing agent, for example, Sn(II), Fe(II), Cu(I), Ti(III) or Sb(III); Sn(II) is excellently suitable. The protein constituent of the above-mentioned kits, i.e. preferably the protein conjugate, may be supplied as a solution, for example, in the form of a physiological saline solution, or in some buffer solution, but is preferably present in a dry state, for example, in the freeze-dried state. When used as a component for an injection liquid it should be sterile, in which, when the constituent is in the dry state, the user should preferably use a sterile physiological saline solution as a solvent. If desired, the above-mentioned constituent may be stabilized in the conventional manner with suitable stabilizers, for example, ascorbic acid, gentisic acid or salts of these acids, or it may comprise other auxiliary agents, for example, fillers, such as glucose, lactose, mannitol, an the like.

The invention will now be described in greater detail with reference to the ensuing specific example.

EXAMPLE

To show the results of purification of human immunoglobulin with the aid of protein-A comprising microorganisms the following experiments are carried out.

To be able to determine the results of the purification procedure, a protein is used, labelled with Tc-99m as described in the Intern. patent application WO 89/07456, mentioned hereinbefore. This product is prepared from a lyophilized kit containing 1 mg 2-iminothiolane-modified polyclonal human immunoglobulin and stannous tartrate, marketed by Mallinckrodt Diagnostica under the (registered) trade name Technescan HIG, and technetium-99m in the form of a pertechnetate solution, obtained from a molybdenumtechnetium generator.

The above labelled human immunoglobulin (Ig) is purified by affinity chromatography by using an ImmunoPure® AIgG purification kit. Such a kit comprises protein-A, immobilized on sepharose. Hereafter 0.17 mg of the purified Ig and 0.17 mg of unpurified Ig are labelled with Tc-99m as described above and then incubated with approx. 5 x $10^8$ CFU of Staphylococcus aureus (Cowan) for 24 h at 37 °C. The activity in both pellet and supernatant is determined after centrifugation. The activity in the pellet compared with that in both the pellet and the supernatant is 88% for the protein-A purified labelled Ig and 46% for the unpurified labelled Ig. The conclusion can be drawn, that there is a direct effect of the protein-A purification on the binding of immunoglobulin to the used micro-organisms.

In a comparable experiment both purified and unpurified Ig are labelled in the presence of $NaBH_4$ (0.5-1 $\mu$g/0.1-1 mg Ig) in an aqueous buffer solution (pH 5.6) and in the presence of Sn(II); incubation at room temperature. In this experiment equally favourable results are obtained.

## Claims

**1.** A method of preparing a diagnostic agent for detecting and locating inflammations in a warm-blooded living being by attaching a detectable label to an immunospecific protein or proteinaceous substance, characterized in that prior to the labelling procedure the protein or proteinaceous substance is purified with the aid of a substance comprising immunoglobulin receptors.

**2.** A method as claimed in claim 1, characterized in that the purification is carried out by immobilizing the substance comprising immunoglobulin receptors on a substrate, and by then subjecting a solution of the protein or proteinaceous substance to be purified to affinity chromatography wherein said substrate is used as the immobile phase.

**3.** A method as claimed in claim 1 or 2, wherein said detectable label is a radionuclide selected from the group consisting of I-123, I-131, Br-75, Br-76, Tc-99m, Pb-203, Ga-67, Ga-68, As-72, In-111, In-113m, Ru-97, Cu-62, Cu-64, Cu-67, Fe-52, Mn-52m and Cr-51.

**4.** A method as claimed in claim 1 or 2, wherein said detectable label is a paramagnetic label selected from the group consisting of F-19, D, Na-23, P-31, Gd-157, Mn-55, Dy-162, Cr-52 and Fe-56.

**5.** A method as claimed in any of the preceding claims, wherein the protein or proteinaceous substance is human immunoglobulin or a suitable fragment thereof, and wherein the purification is carried out with the aid of a substance comprising bacterial immunoglobulin receptors, preferably protein-A.

**6.** A diagnostic agent obtained by using the method as claimed in any of the preceding claims.

**7.** A pharmaceutical composition which, in addition to a pharmaceutically acceptable carrier comprises a diagnostic agent, characterized in that the composition comprises a diagnostic agent obtained by using the method as claimed in any of claims 1-5.

**8.** A method of performing a diagnostic examination, characterized in that a composition as claimed in claim 7, if desired after dilution with a pharmaceutically acceptable liquid, is administered to a warm-blooded living being in a quantity sufficient for externally imaging said being to detect and locate an inflammation in said being.

**9.** A kit for preparing a radiopharmaceutical composition, comprising (i) an immunospecific protein or proteinaceous substance purified as described in claim 1 or 2, to which substance, if desired, an inert pharmaceutically acceptable carrier and/or formulating agents and/or auxiliary substances is/are added, (ii) a solution of a

compound of a radionuclide, and (iii) instructions for use with a prescription for reacting the ingredients present in the kit.

**10.** A kit for preparing a radiopharmaceutical composition, comprising (i) a substance obtained by treating an immunospecific protein or proteinaceous substance, purified as described in claim 1 or 2, with a suitable disulphide-reducing agent, to which substance, if desired, an inert pharmaceutically acceptable carrier and/or formulating agents and/or auxiliary substances is/are added, (ii) a solution of a salt or chelate of a metal-radionuclide, and (iii) instructions for use with a prescription for reacting the ingredients present in the kit.

**11.** A kit for preparing a radiopharmaceutical composition, comprising (i) a substance obtained by treating an immunospecific protein or proteinaceous substance, purified as described in claim 1 or 2, with a suitable disulphide-reducing agent, to which substance, if desired, an inert pharmaceutically acceptable carrier and/or formulating agents and/or auxiliary substances is/are added, (ii) a pertechenate-reducing agent and, if desired, a chelator, said ingredients (i) and (ii) optionally being combined, and (iii) instructions for use with a prescription for reacting the ingredients of the kit with technetium-99m in the form of a pertechnetate solution.

**12.** A kit for preparing a radiopharmaceutical composition, comprising (i) an immunospecific protein or proteinaceous substance purified as described in claim 1 or 2, to which substance, if desired, an inert pharmaceutically acceptable carrier an/or formulating agents and/or auxiliary substances is/are added, (ii) a borohydride, (iii) preferably a pertechnetate-reducing agent and, if desired, a chelator, said ingredients (ii) and (iii) optionally being combined, and (iv) instructions for use with a prescription for reacting the ingredients of the kit with technetium-99m in the form of a pertechnetate solution.

**13.** A kit for preparing a radiopharmaceutical composition, comprising (i) a protein conjugate, obtained by modifying an immunospecific protein or proteinaceous substance, purified as described in claim 1 or 2, by a treatment with a coupling agent, to which protein conjugate, if desired, an inert pharmaceutically acceptable carrier and/or formulating agents and/or auxiliary substances is/are added, (ii) a solution of a salt or chelate of a metal-radionuclide, and (iii) instructions for use with a prescription for reacting the ingredients present in the kit.

**14.** A kit for preparing a radiopharmaceutical composition, comprising (i) a protein conjugate, obtained by modifying an immunospecific protein or proteinaceous substance, purified as described in claim 1 or 2, by a treatment with a coupling agent, to which protein conjugate, if desired, an inert pharmaceutically acceptable carrier and/or formulating agents and/or auxiliary substances is/are added, (ii) a pertechnetate-reducing agent and, if desired, a chelator, said ingredients (i) and (ii) optionally being combined, and (iii) instructions for use with a prescription for reacting the ingredients of the kit with technetium-99m in the form of a pertechnetate solution.

**15.** A kit as claimed in claim 13 or 14, wherein said protein conjugate has been obtained by treating an immunospecific protein or proteinaceous substance, purified as described in claim 1 or 2, with a coupling agent of the general formula

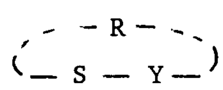

wherein
R is a branched or non-branched, optionally substituted hydrocarbyl radical, which may be interrupted by one or more hetero-atoms selected from N, O and S and/or by one or more NH groups, and
Y is a group which is capable of reacting with a functional group of the protein and which is preferably selected from the group consisting of carbonyl, carbimidoyl, N-($C_1$-$C_6$) alkylcarbimidoyl, N-hydroxycarbimidoyl and N-($C_1$-$C_6$) alkoxycarbimidoyl, or a water-soluble salt of this ring compound.

**16.** A kit as claimed in any of claims 9-15, wherein said immunospecific protein or proteinaceous substance is human immunoglobulin or a suitable fragment thereof, purified as described in claim 5.

European Patent Office

# PARTIAL EUROPEAN SEARCH REPORT

which under Rule 45 of the European Patent Convention shall be considered, for the purposes of subsequent proceedings, as the European search report

Application number

EP 90 20 2996

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | EP-A-0 237 150 (NEORX CORP.) | | A 61 K 49/02 |
| | * Claims 1-8 * | 1,2, 5,9 | |
| A | | 3,6, 7,10 | |
| | -- | | |
| Y | WO-A-90 09 237 (F. ROBERTS) | | |
| | * Whole document * | 1,2, 5,9 | |
| A | | 16 | |
| | -- | | |
| Y | WO-A-87 04 351 (THE GENERAL HOSPITAL CO.) | | |
| | * Claims 1-35 * | 1,2, 5,9 | TECHNICAL FIELDS SEARCHED (Int. Cl.4) |
| A | ./. -- | 3,4, 6,10 | A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7,9-16

Claims searched incompletely:

Claims not searched: 8

Reason for the limitation of the search:

Method for treatment of the human or animal body by surgery or therapy (See art. 52(4) of the European Patent Convention)

| Place of search | Date of completion of the search | Examiner |
|---|---|---|
| THE HAGUE | 19-07-1991 | BERTOCCHI |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number

EP 90 20 2996



| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | EP-A-0 250 013 (MALLINCKRODT INC.)<br>* Pages 1-8; claims 1-8 * | 1-16 | |
| | -- | | |
| A,D | WO-A-89 07 456 (MALLINCKRODT INC.)<br>* Abstract * | 1-16 | |
| | ----- | | |
| | | | TECHNICAL FIELDS SEARCHED (Int Cl 4) |